# FASCICULE DE BREVET EUROPEEN

(11) **EP 1 753 726 B1**
(45) Date de publication et mention de la délivrance du brevet: **06.01.2010**
(21) Numéro de dépôt: 05770985.9
(22) Date de dépôt: 10.05.2005
(51) Int. Cl.: C07D 231/16, C07D 231/22, C07D 231/14

(54) **PROCEDE DE PREPARATION DE DERIVES DE L'ACIDE 1,5-DIPHENYLPYRAZOLE CARBOXYLIQUE**
VERFAHREN ZUR HERSTELLUNG VON 1,5-DIPHENYLPYRAZOLCARBONSÄUREDERIVATEN
METHOD FOR PRODUCING 1,5-DIPHENYLPYRAZOLE CARBOXYLIC ACID DERIVATIVES

(30) Priorité: 10.05.2004 FR 0405057
(43) Date de publication de la demande: 21.02.2007
(73) Titulaire: Sanofi-Aventis, 75013 Paris (FR)
(72) Inventeur: DAUMAS, Marc, F-04700 Oraison (FR); VAYRON, Philippe, FR-05300 Chateauneuf de Chabre (FR)
(74) Mandataire: Kugel, Dominique
(86) Numéro de dépôt international: PCT/FR2005/001155
(87) Numéro de publication internationale: WO 2005/115989

(56) Documents cités:
- EP-A- 0 576 357
- WO-A2-01/40195
- WANG X-J ET AL: "Cross-coupling of 1-aryl-5-bromopyrazoles: regioselective synthesis of 3,5-disubstituted 1-arylpyrazoles" TETRAHEDRON LETTERS, ELSEVIER SCIENCE PUBLISHERS, AMSTERDAM, NL, vol. 41, no. 24, juin 2000 (2000-06), pages 4713-4716, XP004205605 ISSN: 0040-4039
- DATABASE BEILSTEIN BEILSTEIN INSTITUTE FOR ORGANIC CHEMISTRY, FRANKFURT-MAIN, DE; XP002310652 & B.COTTINEAU, J.CHENAULT: SYN. LETT., vol. 5, 2002, pages 769-770,
- REGAN J ET AL: "Pyrazole urea-based inhibitors of p38 MAP kinase: from lead compound to clinical candidate" JOURNAL OF MEDICINAL CHEMISTRY, AMERICAN CHEMICAL SOCIETY. WASHINGTON, US, vol. 45, no. 14, 25 mai 2002 (2002-05-25), pages 2994-3008, XP002243050 ISSN: 0022-2623
- R. D'ALESSIO ET ALL: "Syntesis and Immunosuppressive Activity of Novel Prodigiosin Derivatives" J.MED:CHEM:, vol. 43, no. 13, 2000, pages 2557-2565, XP002348043
- DATABASE CA [Online] CHEMICAL ABSTRACTS SERVICE, COLUMBUS, OHIO, US; NORO, MASAKI: "Silver halide photographic material containing polymethyne pyrazolone dye" XP002348184 extrait de STN Database accession no. 1998:198072 & JP 10 083041 A2 (FUJI PHOTO FILM CO., LTD., JAPAN) 31 mars 1998 (1998-03-31)
- RAIMUNDO, BRIAN C. ET ALL: "Integrating Fragment Assembly and Biophysical in the Chemical Advancement of Small-Molecule Antagonists of IL-2. An Approch for Inhibiting Protein-Protein Interaction" J.MED.CHEM., vol. 47, no. 12, 2004, pages 3111-3130, XP002348124

## Description

La présente invention a pour objet un nouveau procédé pour la préparation de dérivés de l'acide 1,5-diphénylpyrazole-3-carboxylique.

Des esters de l'acide 1,5-diphényl-4-méthylpyrazole-3-carboxylique sont décrits en particulier dans le brevet européen EP 576 357 et le brevet américain US 5 624 941. Ces esters sont des intermédiaires utiles pour la préparation de dérivés de l'acide 1,5-diphényl-4-méthylpyrazole-3-carboxylique qui sont des antagonistes des récepteurs aux cannabinoïdes CB₁.

Plus particulièrement, le 5-(4-chlorophényl)-1-(2,4-dichlorophényl)-4-méthylpyrazol-3-carboxylate d'éthyle, décrit dans le brevet européen EP 656 354, est un intermédiaire utile pour la préparation du N-pipéridino-5-(4-chlorophényl)-1-(2,4-dichlorophényl)-4-méthylpyrazole-3-carboxamide dont la dénomination commune internationale est rimonabant.

La présente invention a pour objet un procédé de préparation d'un composé de formule : 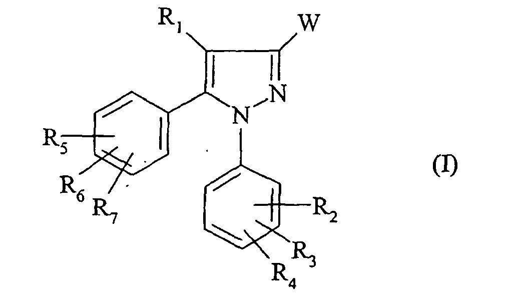 dans laquelle :
- W représente un groupe -COOR ou un radical -CN ;
- R₁ représente un atome d'hydrogène ou d'halogène ou un groupe (C₁-C₄)alkyle ;
- R₂, R₃, R₄, R₅, R₆, R₇ représentent chacun indépendamment l'un de l'autre un atome d'hydrogène ou d'halogène ou un groupe (C₁-C₄)alkyle, (C₁-C₄)alcoxy ou trifluorométhyle ;
- R représente un groupe (C₁-C₄)alkyle ou benzyle ;
**caractérisé en ce que** l'on fait réagir, dans un solvant, en présence d'un catalyseur en milieu basique, un dérivé d'acide phénylboronique de formule : 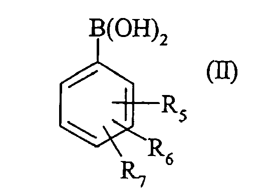 dans laquelle R₅, R₆ et R₇ sont tels que définis pour (I), sur un composé de formule : 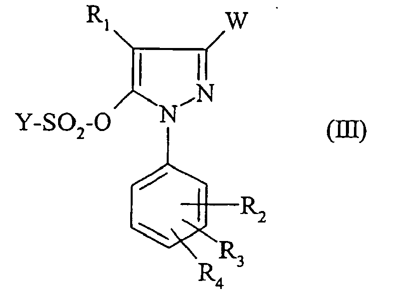 dans laquelle :
- W, R₁, R₂, R₃, R₄ sont tels que définis pour (I) ;
- Y représente un groupe (C₁-C₄)alkyle, (C₁-C₄)perfluoroalkyle, phényle non substitué ou substitué par un groupe méthyle, chloro ou nitro.

Particulièrement, la présente invention a pour objet un procédé de préparation d'un composé de formule : 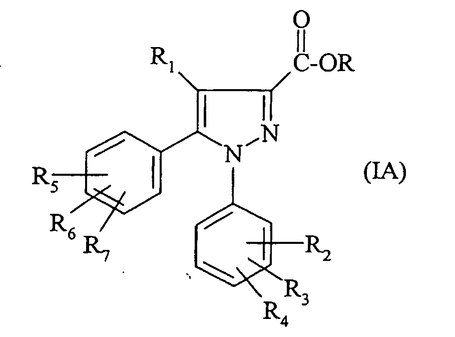 dans laquelle :
- R₁, R₂, R₃, R₄, R₅, R₆, R₇ et R sont tels que définis pour un composé de formule (I) ;
**caractérisé en ce que** l'on fait réagir, dans un solvant, en présence d'un catalyseur en milieu basique, un dérivé d'acide phénylboronique de formule (II) tel que défini ci-dessus sur un composé de formule : 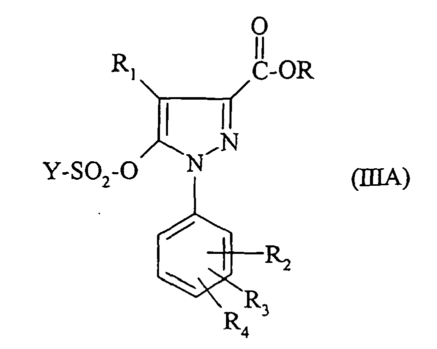 dans laquelle :
- Y, R, R₁, R₂, R₃ et R₄ sont tels que définis ci-dessus.

Particulièrement aussi, la présente invention a pour objet un procédé de préparation d'un composé de formule : 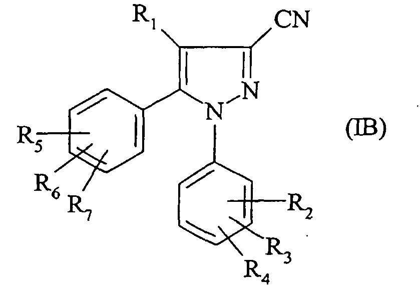 dans laquelle :
- R₁, R₂, R₃, R₄, R₅, R₆ et R₇ sont tels que définis pour un composé de formule (I) ;
**caractérisé en ce que** l'on fait réagir, dans un solvant, en présence d'un catalyseur en milieu basique, un dérivé d'acide phénylboronique de formule (II) tel que défini ci-dessus sur un composé de formule : 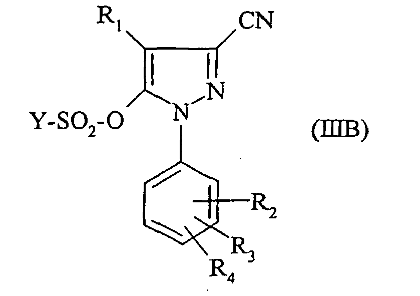 dans laquelle :
- Y, R₁, R₂, R₃ et R₄ sont tels que définis ci-dessus.

Préférentiellement, la réaction d'un composé de formule (II) sur un composé de formule (III), (IIIA) ou (IIIB) est effectuée dans un solvant aromatique, par exemple le toluène, ou dans un solvant éthéré, par exemple le tétrahydrofurane ou le diméthoxyéthane ou dans le dioxane, en présence d'un complexe du palladium, par exemple, le tétrakis(triphénylphosphine)palladium et dans un milieu basique, par exemple en présence d'un carbonate alcalin, tel que le carbonate de sodium ou le carbonate de potassium.

Selon une forme de réalisation préférée, la réaction est effectuée en milieu biphasique, en présence de tétrakis(triphénylphosphine)palladium, le milieu basique étant constitué de carbonate de sodium en solution aqueuse.

Selon un procédé préféré pour la préparation d'un composé de formule (I), on fait réagir un dérivé d'acide phénylboronique de formule (II) sur un composé de formule (III) dans laquelle Y représente un groupe CF₃, à savoir un composé de formule :

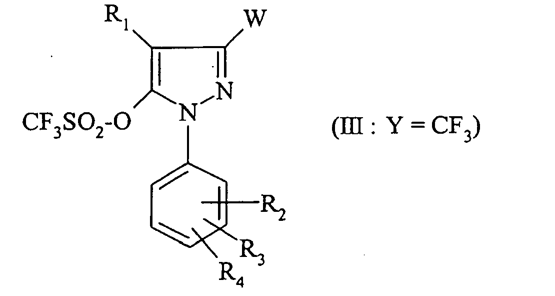

Selon, aussi, un procédé préféré pour la préparation d'un composé de formule (IA), on fait réagir un dérivé d'acide phénylboronique de formule (II) sur un composé de formule (IIIA) dans laquelle Y représente un groupe CF₃, à savoir un composé de formule :

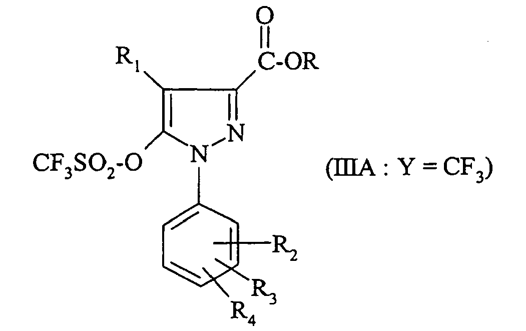

Selon, enfin, un procédé préféré pour la préparation d'un composé de formule (IB), on fait réagir un dérivé d'acide phénylboronique de formule (II) sur un composé de formule (IIIB) dans laquelle Y représente un groupe CF₃, à savoir un composé de formule :

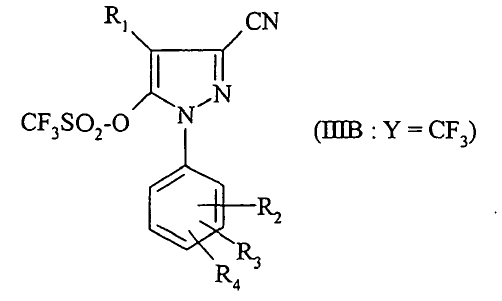

Plus préférentiellement, on fait réagir l'acide 4-chlorophénylboronique sur un composé de formule :

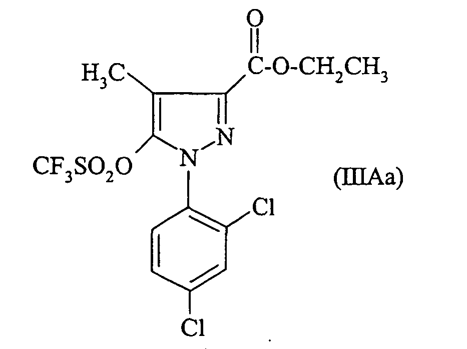

Plus préférentiellement aussi, on fait réagir l'acide 4-bromophénylboronique sur un composé de formule :

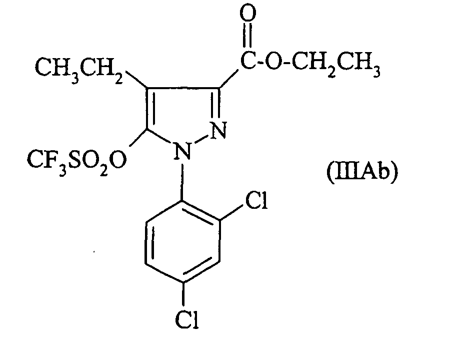

Plus préférentiellement encore, on fait réagir l'acide 4-chlorophénylboronique sur un composé de formule :

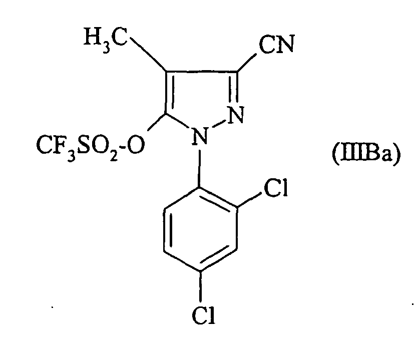

Plus préférentiellement enfin, on fait réagir l'acide 4-bromophénylboronique sur un composé de formule :

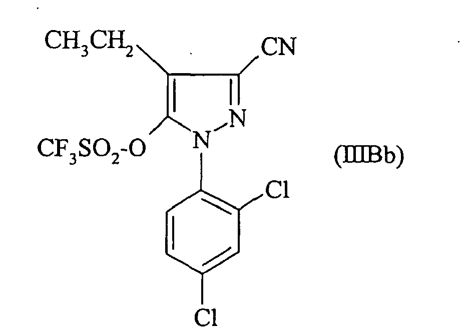

Le composé de formule (III) est obtenu à partir d'un dérivé de pyrazolone de formule :

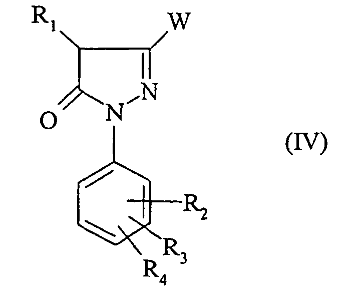

Particulièrement, le composé de formule (IIIA) est obtenu à partir d'un dérivé de pyrazolone de formule :

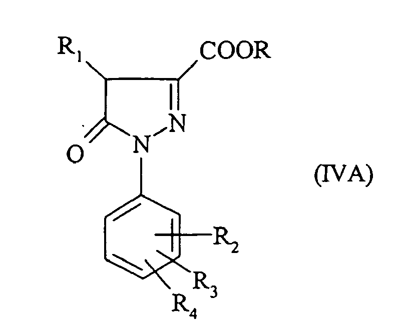

Particulièrement, aussi, le composé de formule (IIIB) est obtenu à partir d'un dérivé de pyrazolone de formule :

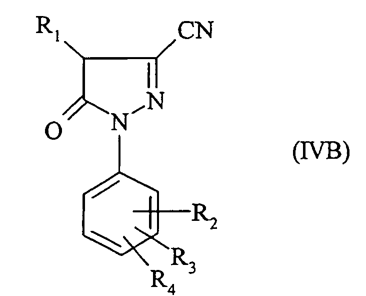

Pour préparer un composé de formule (III), (IIIA) ou (IIIB), on fait réagir sur un composé de formule (IV), (IVA) ou (IVB), un anhydride de formule (YSO₂)₂O ou un chlorure de formule YSO₂Cl ; la réaction étant effectuée en présence d'une base, préférentiellement une amine tertiaire.

De façon particulière, le composé de formule : 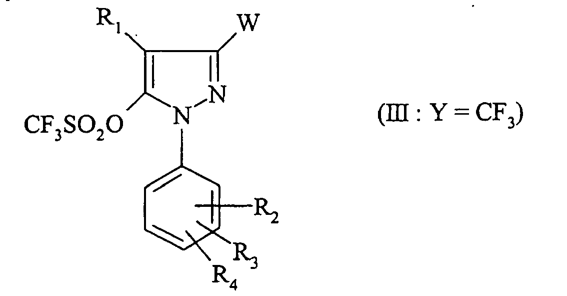 est obtenu par réaction de l'anhydride triflique sur un dérivé de pyrazolone de formule : 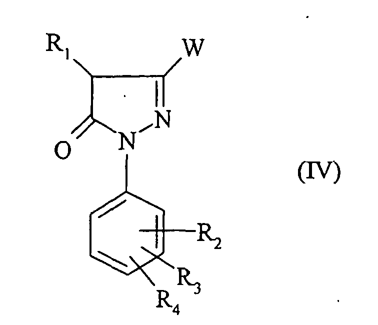 en présence d'une base, telle qu'une amine tertiaire, et dans un solvant, tel que le dichlorométhane.

De façon particulière aussi, le composé de formule : 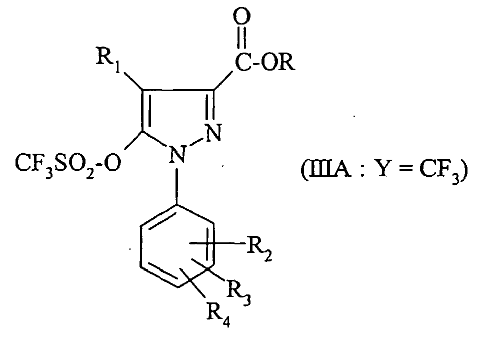 est obtenu par réaction de l'anhydride triflique sur un dérivé de pyrazolone de formule : 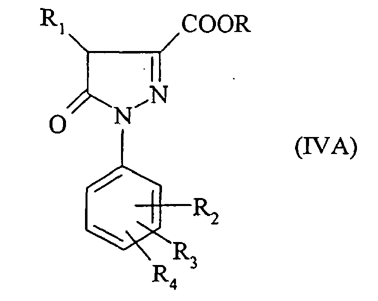 en présence d'une base telle qu'une amine tertaire et dans un solvant tel que le dichlorométhane.

De façon particulière enfin, le composé de formule : 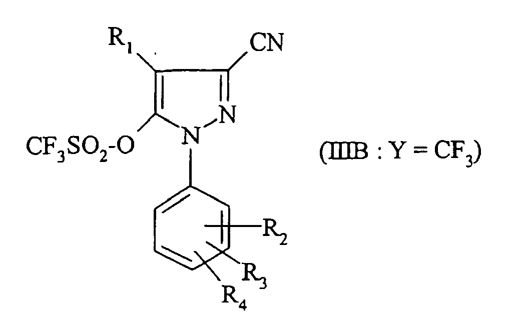 est obtenu par réaction de l'anhydride triflique sur un dérivé de pyrazole de formule : 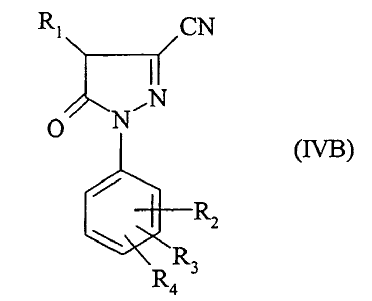 en présence d'une base telle qu'une amine tertiaire et dans un solvant tel que le dichlorométhane.

Préférentiellement, le composé de formule (III), (IIIA) ou (IIIB), dans laquelle Y représente CF₃ est obtenu par action de l'anhydride triflique et de la triéthylamine en mélange équimoléculaire, dans le dichlorométhane, à une température comprise entre -5°C et +5°C.

Le composé de formule : 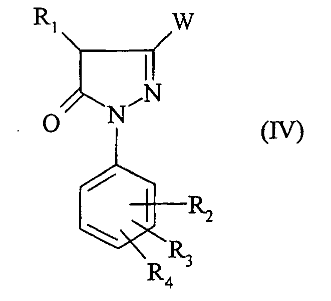 est obtenu par un réaction d'un dérivé d'hydrazine de formule : 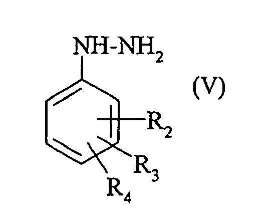 dans laquelle R₂, R₃, R₄ sont tels que définis pour (I) sur un dérivé de formule : 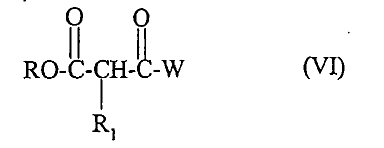 dans laquelle R , R₁ et W sont tels que définis ci-dessus pour (I).

De façon particulière, le composé de formule : 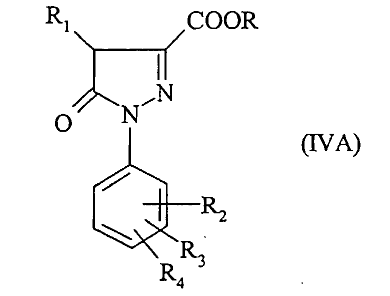 est obtenu par réaction d'un dérivé d'hydrazine de formule (V) sur un dérivé de formule :

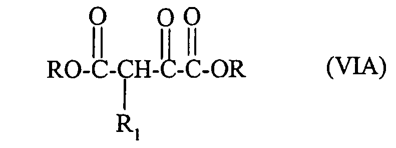

De façon particulière aussi, le composé de formule : 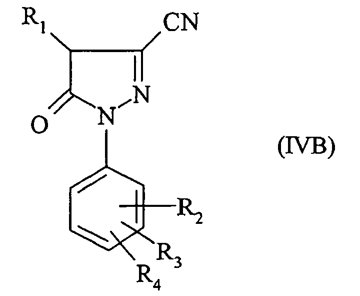 est obtenu par réaction d'un dérivé d'hydrazine de formule (V) sur un dérivé de formule :

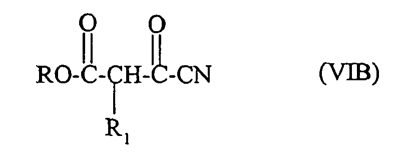

Préférentiellement, on utilise un chlorhydrate de l'hydrazine de formule (V), dans un solvant, en milieu acide, par exemple dans l'acide acétique ; ou dans le toluène en présence d'acide chlorhydrique, acétique ou trifluoroacétique.

Les dérivés de 3-oxosuccinate de formule (VI), (VIA) ou (VIB) sont connus ou préparés par des méthodes connues telles que la condensation de Claisen d'un énolate d'ester sur un ester d'acide oxalique.

Le composé de formule (IV) peut exister sous 2 formes tautomères ;

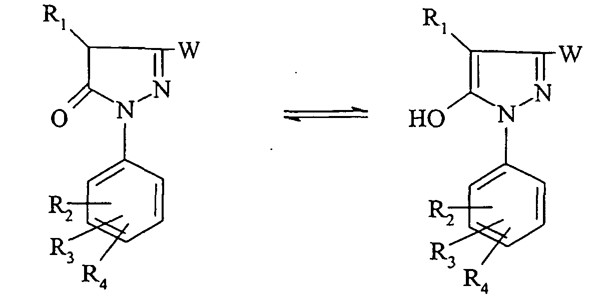

Selon un autre de ses aspects, la présente invention a pour objet les composés de formule : 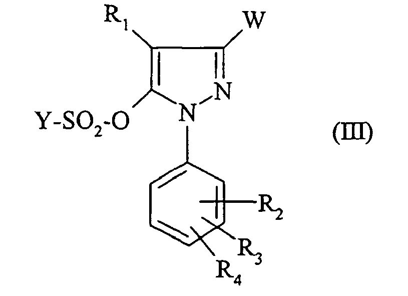 dans laquelle :
- W représente un groupe -COOR ou un radical CN ;
- Y représente un groupe (C₁-C₄)alkyle, (C₁-C₄)perfluoroalkyle, phényle non substitué ou substitué par un groupe méthyle, chloro ou nitro ;
- R₁ représente un atome d'hydrogène ou d'halogène ou un groupe (C₁-C₄)alkyle;
- R₂, R₃, R₄ représentent chacun indépendamment l'un de l'autre un atome d'hydrogène ou d'halogène ou un groupe (C₁-C₄)alkyle, (C₁-C₄)alcoxy, trifluorométhyle ;
- R représente un groupe (C₁-C₄)alkyle ou benzyle ;

Préférentiellement, la présente invention a pour objet les composés de formule : 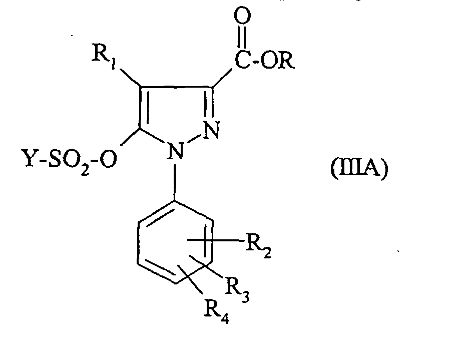 dans laquelle :
- Y, R, R₁, R₂, R₃ et R₄ sont tels que définis ci-dessus.

Préférentiellement, la présente invention a pour objet les composés de formule : 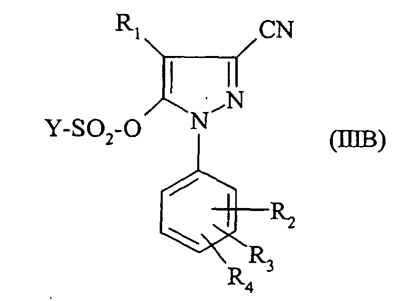 dans laquelle :
- Y, R₁, R₂, R₃ et R₄ sont tels que définis ci-dessus.

Plus préférentiellement, la présente invention a pour objet le composé de formule :

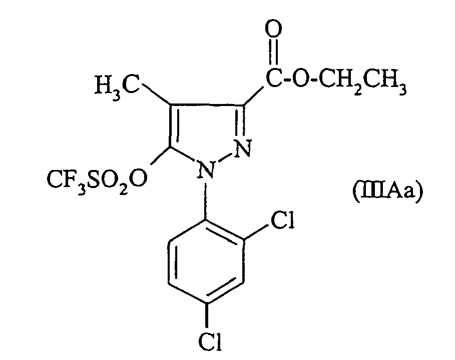

Plus préférentiellement aussi, la présente invention a pour objet le composé de formule :

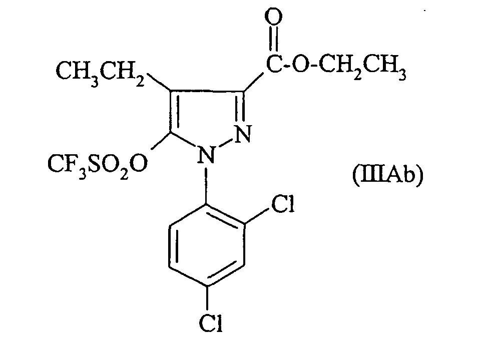

Plus préférentiellement encore, la présente invention a pour objet le composé de formule :

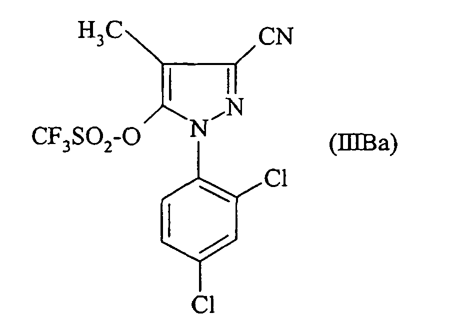

Plus préférentiellement enfin, la présente invention a pour objet un composé de formule :

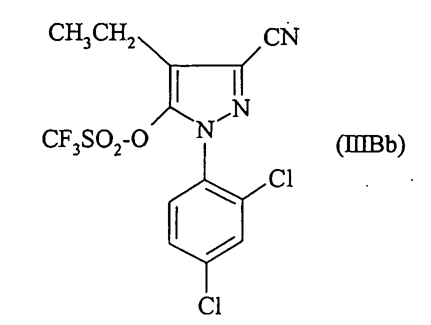

L'exemple qui suit n'est pas limitatif, il illustre un mode de réalisation de l'invention.

Les spectres de masse sont mesurés en mode d'ionisation dit Electrospray (ES).

Les signaux observés en RMN sont exprimés ainsi : s: singulet ; se : singulet élargi ; d : doublet ; d.d : doublet dédoublé ; t : triplet ; td : triplet dédoublé ; q : quadruplet ; m : massif ; mt : multiplet.

### EXEMPLE 1

Préparation du 5-(4-chlorophényl)-1-(2,4-dichlorophényl)-4-méthyl-1*H*-pyrazole-3-carboxylate d'éthyle.

### A) 1-(2,4-Dichlorophényl)-4-méthyl-5-oxo-4,5-dihydro-1H-pyrazole-3-carboxylate d'éthyle.

On place sous azote 12,6 g de chlorhydrate de 2,4-dichlorophénylhydrazine dans 100 ml de toluène ; après agitation, on ajoute 10 g de 2-méthyl-3-oxosuccinate de diéthyle puis on chauffe et on ajoute à 55°C 5 ml de TFA. On laisse sous agitation, à reflux du solvant pendant 4 heures et demie. On laisse revenir à TA puis on chauffe à 75°C et on hydrolyse le milieu réactionnel par 30 ml d'eau. On décante, la phase aqueuse est écartée puis la phase organique est évaporée pour éliminer le TFA résiduel. On reprend la phase organique par 10 ml de toluène puis le produit attendu cristallise, m = 10,2 g.
ES⁻ : [M-H]⁻= 313,0
RMN (DMSO-d6 1H à 300 MHz) : 1,26 ppm : t : 3H ; 2,11 ppm : s : 3H ; 4,23 ppm : q : 2H ; 7,57 ppm : m : 2H ; 7,87 ppm : se : 1H ; 11,04 ppm : se : 1H.

### B) 1-(2,4-Dichlorophényl)-4-méthyl-5-(((trifluorométhyl)sulfonyl)oxy)-1H-pyrazole-3-carboxylate d'éthyle.

Sous azote, on met en suspension 5 g de pyrazolone obtenue à l'étape précédente dans 25 ml de DCM et l'on refroidit à 0°C sous agitation. On ajoute 2,4 ml de TEA puis 3 ml d'anhydride triflique et on maintient l'agitation à 0°C pendant 15 minutes. Le milieu réactionnel est hydrolysé par 20 ml de DCM. On décante le milieu réactionnel puis la phase organique est lavée par 20 ml d'eau. On écarte la phase aqueuse. La phase organique est évaporée et l'huile obtenue est chromatographiée sur silice en éluant par un mélange pentane/AcOEt (90/10 ; v/v). Les fractions contenant le composé attendu sont rassemblées et évaporées à sec. On obtient 6,77 g du composé attendu.
ES⁺ : [M+Na]⁺= 468,8
RMN (DMSO-d6 1H à 300 MHz) : 1,31 ppm : t : 3H ; 2,27 ppm : s : 3H ; 4,34 ppm : q : 2H ; 7,72 ppm : dd : 1H ; 7,79 ppm : d : 1H ; 8,04 ppm : d : 1H.

### C) 5-(4-chlorophényl)-1-(2,4-dichlorophényl)-4-méthyl-1H-pyrazole-3-carboxylate d'éthyle.

Sous azote, on mélange 2,55 g du triflate de pyrazole de l'étape précédente, 1,08 g d'acide 4-chlorophénylboronique et 67 mg de tétrakis(triphénylphosphine)palladium ; on ajoute 25 ml de toluène et 7,1 ml d'une solution aqueuse de carbonate de sodium 2M. Le milieu réactionnel est agité à 65°C pendant 6 heures. Le milieu réactionnel est décanté, la phase aqueuse est écartée puis la phase organique est lavée par 10 ml d'eau. Après décantation, la phase organique est évaporée. Le produit obtenu est purifié par chromatographie sur silice en éluant par un mélange cyclohexane/AcOEt (85/15 ; v/v). On obtient 1,890 g du composé attendu.

RMN (DMSO-d6 1H à 300 MHz) : 1,31 ppm : t : 3H ; 2,23 ppm : s : 3H ; 4,32 ppm : q : 2H ; 7,24 ppm : d : 2H ; 7,46 ppm : d : 2H ; 7,57 ppm : dd : 1H ; 7,73 ppm : d : 1H ; 7,77 : d : 1H.

## Revendications

1. Procédé de préparation d'un composé de formule : 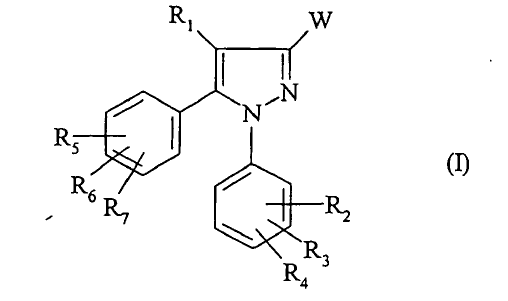 dans laquelle :
- W représente un groupe -COOR ou un radical -CN ;
- R₁ représente un atome d'hydrogène ou d'halogène ou un groupe (C₁-C₄)alkyle ;
- R₂, R₃, R₄, R₅, R₆, R₇ représentent chacun indépendamment l'un de l'autre un atome d'hydrogène ou d'halogène ou un groupe (C₁-C₄)alkyle, (C₁-C₄)alcoxy, trifluorométhyle ;
- R représente un groupe (C₁-C₄)alkyle ou benzyle ;
**caractérisé en ce que** l'on fait réagir un dérivé d'acide phénylboronique de formule : 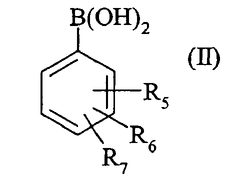 dans laquelle R₅, R₆, R₇ sont tels que définis ci-dessus pour (I) sur un composé de formule : 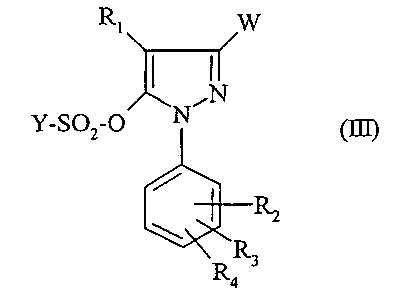 dans laquelle :
- W, R₁, R₂, R₃, R₄ sont tels que définis ci-dessus pour (I) ;
- Y représente un groupe (C₁-C₄)alkyle, (C₁-C₄)perfluoroalkyle, phényle non substitué ou substitué par un groupe méthyle, chloro ou nitro.

2. Procédé selon la revendication 1, de préparation d'un composé de formule : 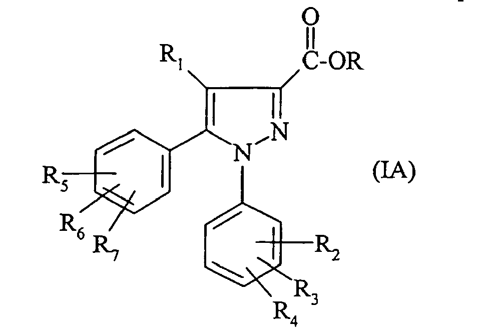 dans laquelle :
- R₁, R₂, R₃, R₄, R₅, R₆, R₇ et R sont tels que définis pour un composé de formule (I) à la revendication 1 ;
**caractérisé en ce que** l'on fait réagir un dérivé d'acide phénylboronique de formule (II) tel que défini à la revendication 1 sur un composé de formule : 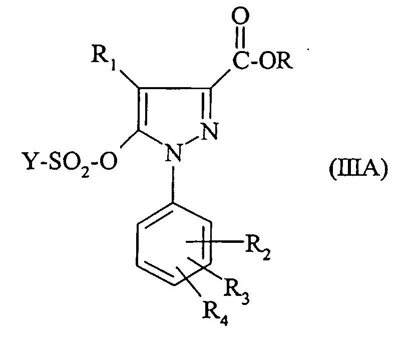 dans laquelle :
- Y, R₁, R₂, R₃ et R₄ sont tels que définis à la revendication 1.

3. Procédé selon la revendication 1, de préparation d'un composé de formule : 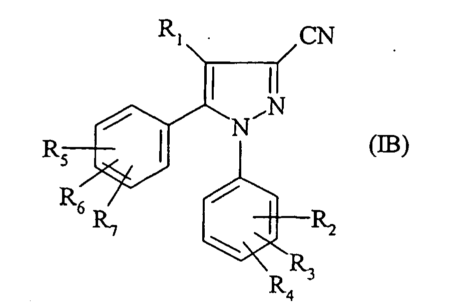 dans laquelle :
- R₁, R₂, R₃, R₄, R₅, R₆ et R₇ sont tels que définis pour un composé de formule (I) à la revendication 1 ;
**caractérisé en ce que** l'on fait réagir un dérivé d'acide phénylboronique de formule (II) tel que défini à la revendication 1 sur un composé de formule : 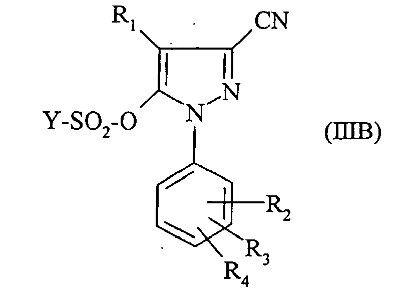
dans laquelle :
- Y, R, R₁, R₂, R₃ et R₄ sont tels que définis à la revendication 1.

4. Procédé selon la revendication 1 **caractérisé en ce que** on fait réagir un dérivé d'acide phénylboronique de formule (II) tel que défini à la revendication 1 sur un composé de formule : 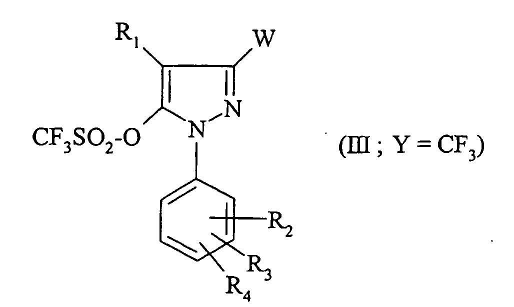 dans laquelle :
- W, R₁, R₂, R₃, R₄ sont tels que définis à la revendication 1.

5. Procédé selon la revendication 2 **caractérisé en ce que** on fait réagir un dérivé d'acide phénylboronique de formule (II) tel que défini à la revendication 1 sur un composé de formule : 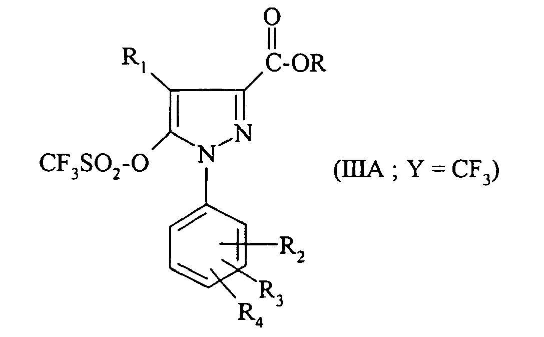 dans laquelle :
- R, R₁, R₂, R₃, R₄ sont tels que définis à la revendication 1.

6. Procédé selon la revendication 3 **caractérisé en ce que** on fait réagir un dérivé d'acide phénylboronique de formule (II) tel que défini à la revendication 1 sur un composé de formule : 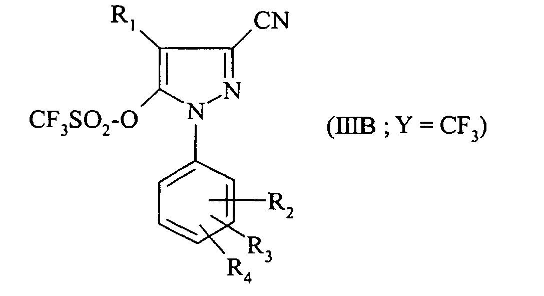 dans laquelle :
- R₁, R₂, R₃, et R₄ sont tels que définis à la revendication 1.

7. Procédé selon la revendication 5 **caractérisé en ce que** on fait réagir l'acide 4-chlorophénylboronique sur un composé de formule :

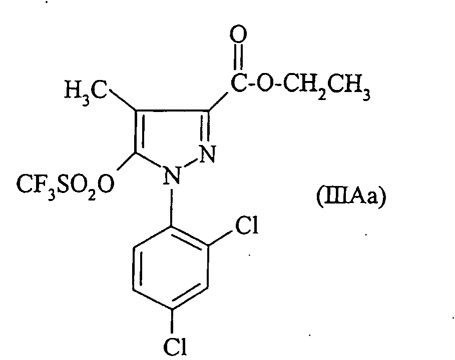

8. Procédé selon la revendication 5 **caractérisé en ce que** on fait réagir l'acide 4-bromophénylboronique sur un composé de formule :

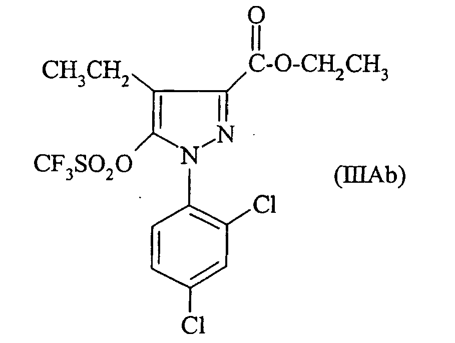

9. Procédé selon la revendication 6 **caractérisé en ce que** on fait réagir l'acide 4-chlorophénylboronique sur un composé de formule :

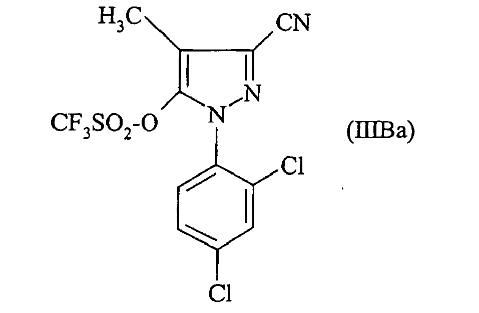

10. Procédé selon la revendication 6 **caractérisé en ce que** on fait réagir l'acide 4-bromophénylboronique sur un composé de formule :

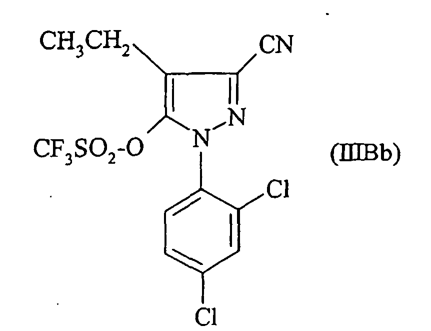

11. Composé de formule : 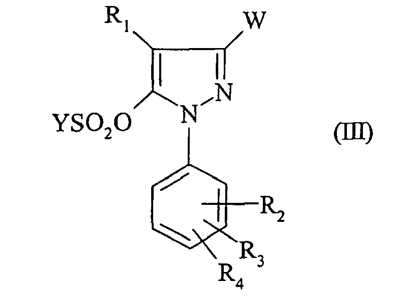 dans laquelle :
- W représente un groupe -COOR ou un radical CN ;
- Y représente un groupe (C₁-C₄)alkyle, (C₁-C₄)perfluoroalkyle, phényle non substitué ou substitué par un groupe méthyle, chloro ou nitro ;
- R₁ représente un atome d'hydrogène ou d'halogène ou un groupe (C₁-C₄)alkyle;
- R₂, R₃, R₄ représentent chacun indépendamment l'un de l'autre un atome d'hydrogène ou d'halogène ou un groupe (C₁-C₄)alkyle, (C₁-C₄)alcoxy, trifluorométhyle ;
- R représente un groupe (C₁-C₄)alkyle ou benzyle.

12. Composé selon la revendication 11 de formule : 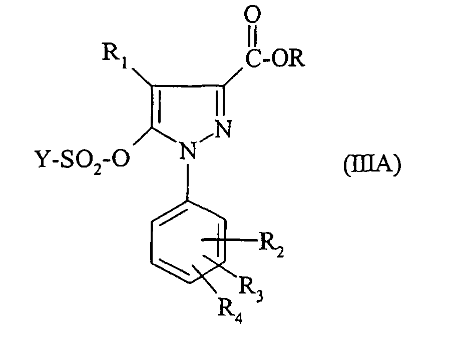 dans laquelle :
- Y, R, R₁, R₂, R₃ et R₄ sont tels que définis à la revendication 11.

13. Composé selon la revendication 11 de formule : 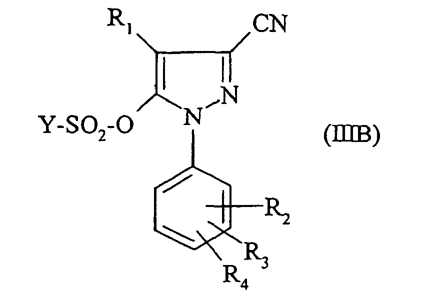 dans laquelle :
- Y, R₁, R₂, R₃ et R₄ sont tels que définis à la revendication 11.

14. Composé selon la revendication 12 de formule :

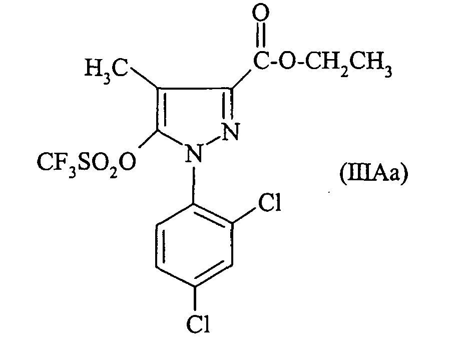

15. Composé selon la revendication 12 de formule :

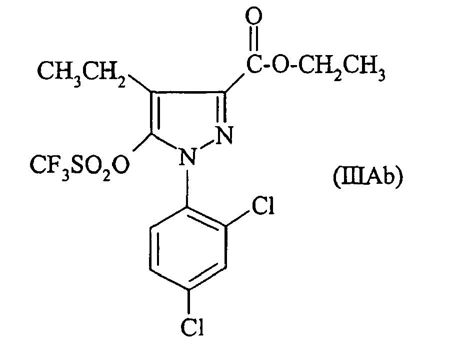

16. Composé selon la revendication 13 de formule :

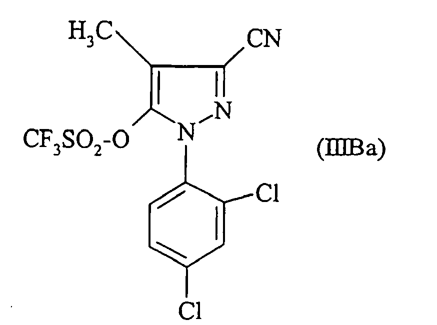

17. Composé selon la revendication 13 de formule :

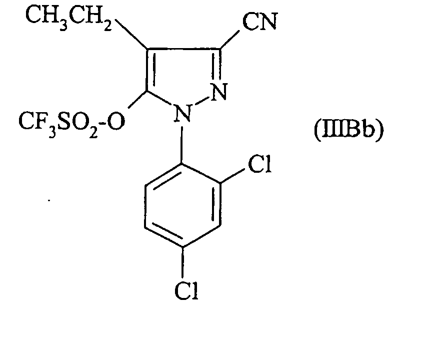

## Claims

1. Process for preparing a compound of formula: 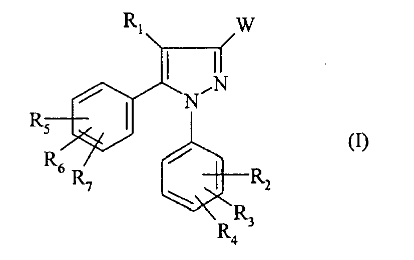 in which:
- W represents a group -COOR or a radical -CN;
- R₁ represents a hydrogen or halogen atom or a (C₁-C₄)alkyl group;
- R₂, R₃, R₄, R₅, R₆ and R₇ each represent, independently of one another a hydrogen or halogen atom or a (C₁-C₄)alkyl, (C₁-C₄)alkoxy or trifluoromethyl group;
- R represents a (C₁-C₄)alkyl or benzyl group;
**characterized in that** a phenylboronic acid derivative of formula: 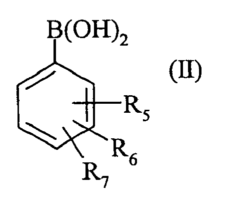 in which R₅, R₆ and R₇ are as defined above for (I), is reacted with a compound of formula: 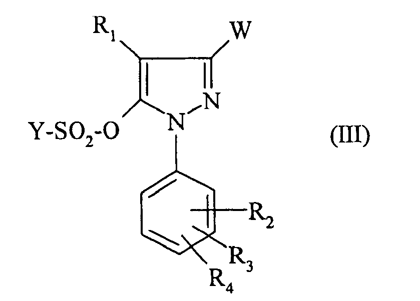 in which:
- W, R₁, R₂, R₃ and R₄ are as defined above for (I);
- Y represents a (C₁-C₄)alkyl group, a (C₁-C₄)perfluoroalkyl group or a phenyl group that is unsubstituted or substituted with a methyl, chloro or nitro group.

2. Process according to Claim 1, for preparing a compound of formula: 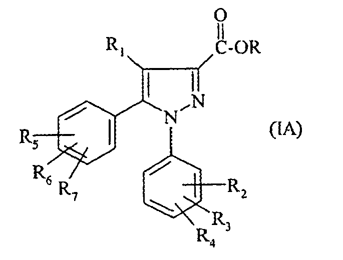 in which:
- R₁, R₂, R₃, R₄, R₅, R₆, R₇ and R are as defined for a compound of formula (I) in Claim 1;
**characterized in that** a phenylboronic acid derivative of formula (II) as defined in Claim 1 is reacted with a compound of formula: 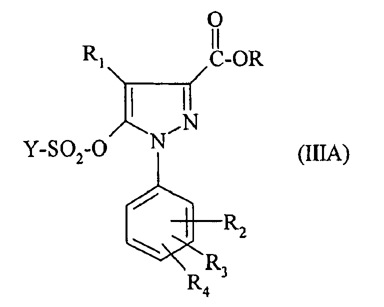 in which:
- Y, R₁, R₂, R₃ and R₄ are as defined in Claim 1.

3. Process according to Claim 1, for preparing a compound of formula: 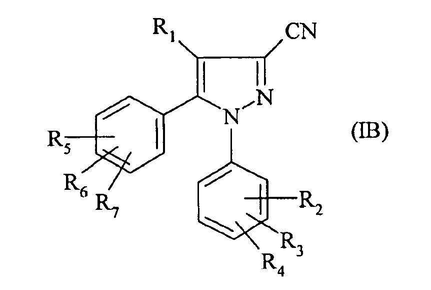 in which
- R₁, R₂, R₃, R₄, R₅, R₆ and R₇ are as defined for a compound of formula (I) in Claim 1;
**characterized in that** a phenylboronic acid derivative of formula (II) as defined in Claim 1 is reacted with a compound of formula: 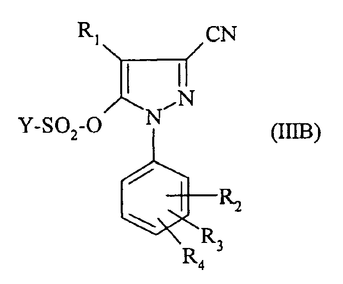 in which:
- Y, R, R₁, R₂, R₃ and R₄ are as defined in Claim 1.

4. Process according to Claim 1,
**characterized in that** a phenylboronic acid derivative of formula (II) as defined in Claim 1 is reacted with a compound of formula: 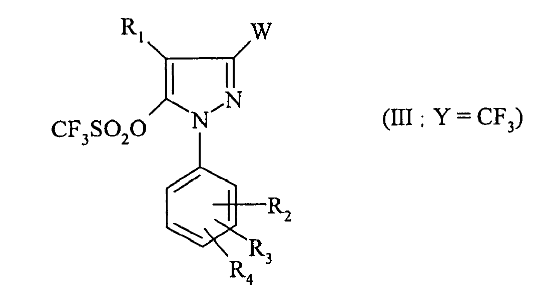 in which:
- W, R₁, R₂, R₃ and R₄ are as defined in Claim 1.

5. Process according to Claim 2,
**characterized in that** a phenylboronic acid derivative of formula (II) as defined in Claim 1 is reacted with a compound of formula: 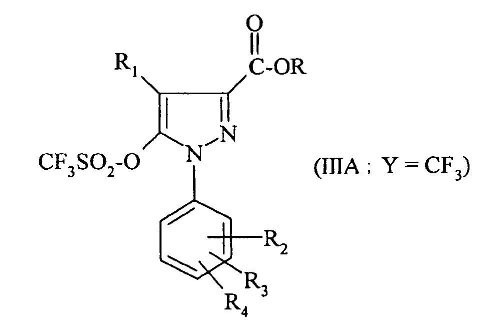 in which:
- R, R₁, R₂, R₃ and R₄ are as defined in Claim 1.

6. Process according to Claim 3,
**characterized in that** a phenylboronic acid derivative of formula (II) as defined in Claim 1 is reacted with a compound of formula: 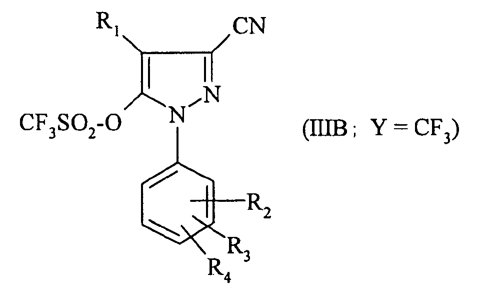 in which:
- R₁, R₂, R₃ and R₄ are as defined in Claim 1.

7. Process according to Claim 5,
**characterized in that** 4-chlorophenylboronic acid is reacted with a compound of formula:

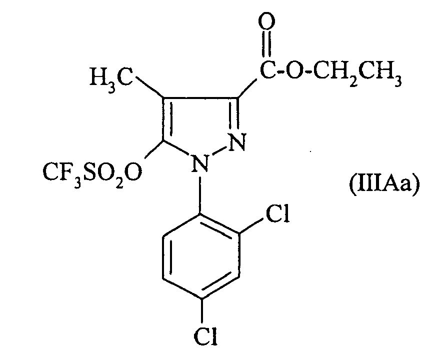

8. Process according to Claim 5,
**characterized in that** 4-bromophenylboronic acid is reacted with a compound of formula:

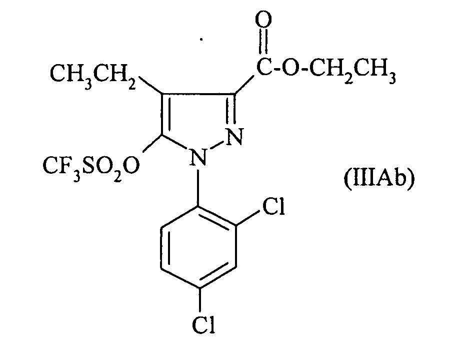

9. Process according to Claim 6,
**characterized in that** 4-chlorophenylboronic acid is reacted with a compound of formula:

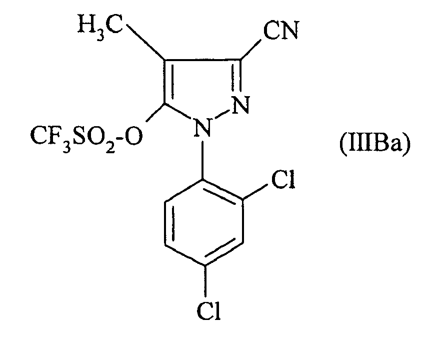

10. Process according to Claim 6,
**characterized in that** 4-bromophenylboronic acid is reacted with a compound of formula:

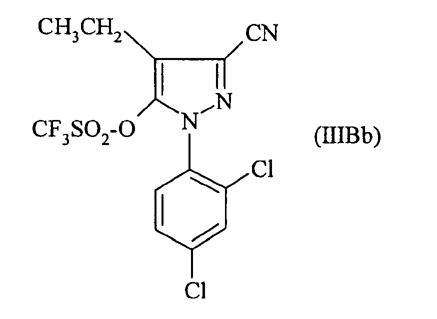

11. Compound of formula: 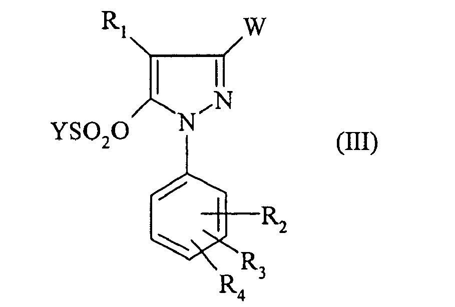 in which:
- W represents a group -COOR or a radical -CN;
- Y represents a (C₁-C₄)alkyl group, a (C₁-C₄)perfluoroalkyl group or a phenyl group that is unsubstituted or substituted with a methyl, chloro or nitro group;
- R₁ represents a hydrogen or halogen atom or a (C₁-C₄)alkyl group;
- R₂, R₃ and R₄ each represent, independently of one another, a hydrogen or halogen atom or a (C₁-C₄)alkyl, (C₁-C₄)alkoxy or trifluoromethyl group;
- R represents a (C₁-C₄)alkyl or benzyl group.

12. Compound according to Claim 11, of formula: 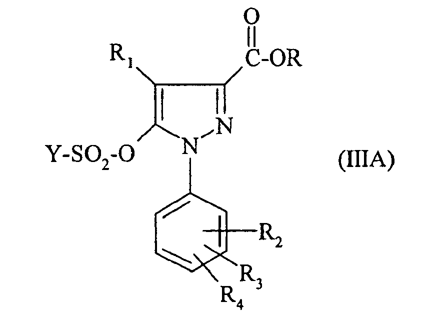 in which:
- Y, R, R₁, R₂, R₃ and R₄ are as defined in Claim 11.

13. Compound according to Claim 11, of formula: 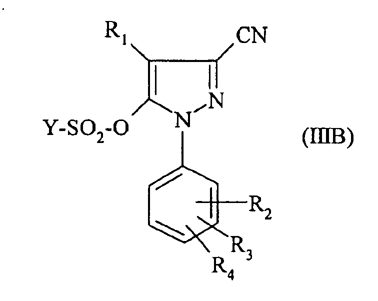 in which:
- Y, R₁, R₂, R₃ and R₄ are as defined in Claim 11.

14. Compound according to Claim 12, of formula:

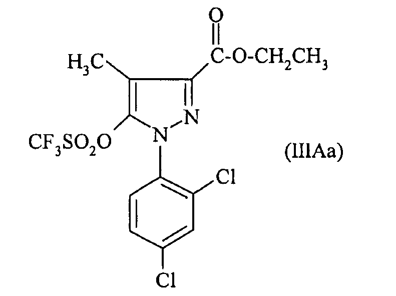

15. Compound according to Claim 12, of formula:

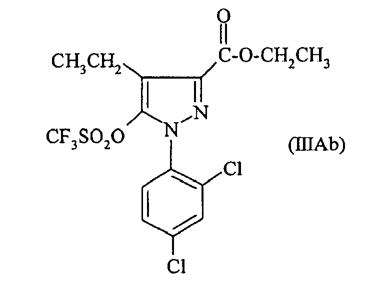

16. Compound according to Claim 13, of formula:

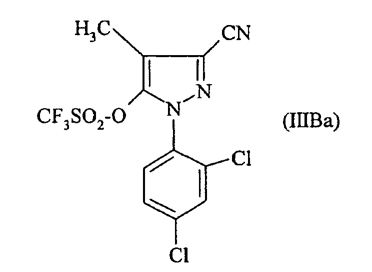

17. Compound according to Claim 13, of formula:

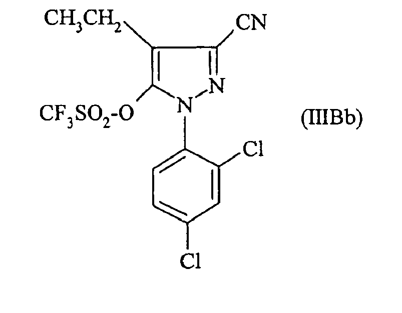

## Patentansprüche

1. Verfahren zur Herstellung einer Verbindung der Formel: 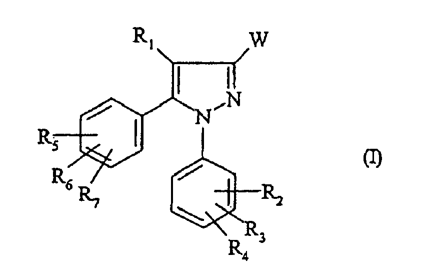 worin:
- W für eine -COOR-Gruppe oder einen -CN-Rest steht;
- R₁ für ein Wasserstoff- oder Halogenatom oder eine (C₁-C₄)-Alkylgruppe steht;
- R₂, R₃, R₄, R₅, R₆ und R₇ jeweils unabhängig voneinander für ein Wasserstoff- oder Halogenatom oder eine (C₁-C₄)-Alkyl-, (C₁-C₄)-Alkoxy- oder Trifluormethylgruppe stehen;
- R für eine (C₁-C₄)-Alkyl- oder Benzylgruppe steht;
**dadurch gekennzeichnet, daß** man ein Phenylboronsäurederivat der Formel: 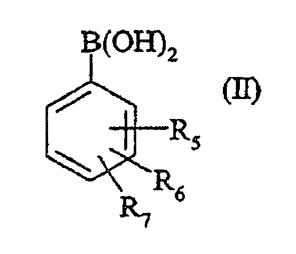 worin R₅, R₆ und R₇ die oben für (I) angegebene Bedeutung besitzen, mit einer Verbindung der Formel: 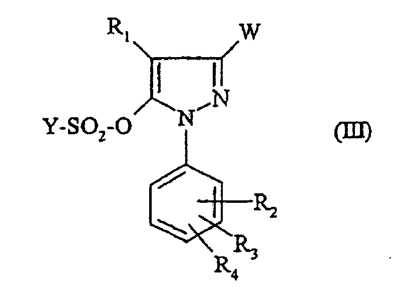 worin:
- W, R₁, R₂, R₃ and R₄ die oben für (I) angegebene Bedeutung besitzen;
- Y für eine (C₁-C₄)-Alkylgruppe, eine (C₁-C₄)-Perfluoralkylgruppe oder eine Phenylgruppe, die gegebenenfalls durch eine Methyl-, Chlor- oder Nitrogruppe substituiert ist, steht;
umsetzt.

2. Verfahren nach Anspruch 1 zur Herstellung einer Verbindung der Formel: 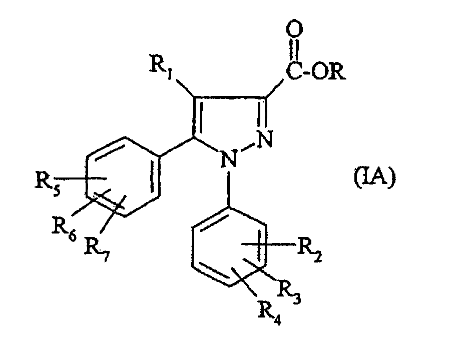 worin:
- R₁, R₂, R₃, R₄, R₅, R₆, R₇ and R die für eine Verbindung der Formel (I) in Anspruch 1 angegebene Bedeutung besitzen;
**dadurch gekennzeichnet, daß** man ein Phenylboronsäurederivat der Formel (II) gemäß Anspruch 1 mit einer Verbindung der Formel: 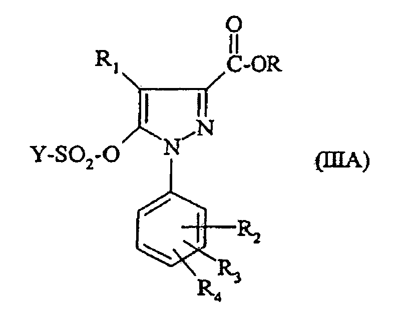 worin:
- Y, R₁, R₂, R₃ and R₄ die oben in Anspruch 1 angegebene Bedeutung besitzen;
umsetzt.

3. Verfahren nach Anspruch 1 zur Herstellung einer Verbindung der Formel: 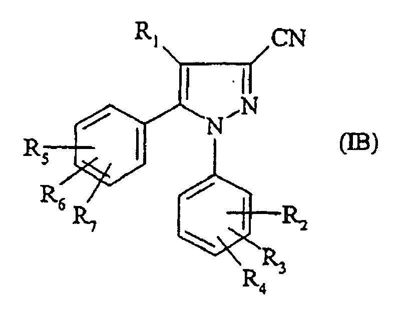 worin:
- R₁, R₂, R₃, R₄, R₅, R₆ und R₇ die für eine Verbindung der Formel (I) in Anspruch 1 angegebene Bedeutung besitzen;
**dadurch gekennzeichnet, daß** man ein Phenylboronsäurederivat der Formel (II) gemäß Anspruch 1 mit einer Verbindung der Formel: 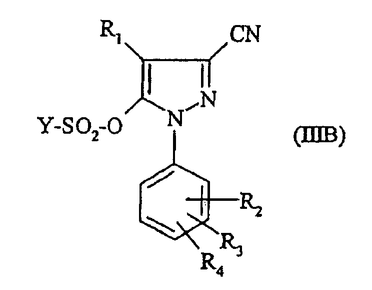 worin:
- Y, R, R₁, R₂, R₃ und R₄ die oben in Anspruch 1 angegebene Bedeutung besitzen;
umsetzt.

4. Verfahren nach Anspruch 1, **dadurch gekennzeichnet, daß** man ein Phenylboronsäurederivat der Formel (II) gemäß Anspruch 1 mit einer Verbindung der Formel: 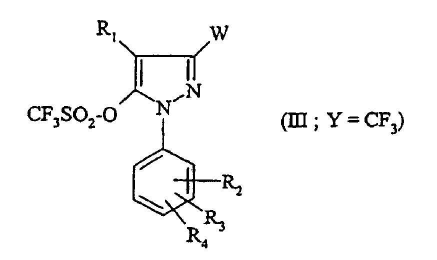 worin:
- W, R₁, R₂, R₃ und R₄ die oben in Anspruch 1 angegebene Bedeutung besitzen;
umsetzt.

5. Verfahren nach Anspruch 2, **dadurch gekennzeichnet, daß** man ein Phenylboronsäurederivat der Formel (II) gemäß Anspruch 1 mit einer Verbindung der Formel: 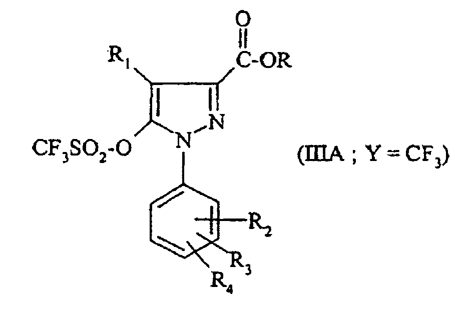 worin:
- R, R₁, R₂, R₃, R₄ die oben in Anspruch 1 angegebene Bedeutung besitzen;
umsetzt.

6. Verfahren nach Anspruch 3, **dadurch gekennzeichnet, daß** man ein Phenylboronsäurederivat der Formel (II) gemäß Anspruch 1 mit einer Verbindung der Formel: 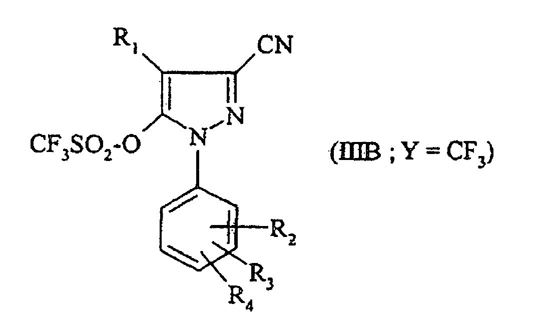 worin:
- R₁, R₂, R₃ und R₄ die oben in Anspruch 1 angegebene Bedeutung besitzen;
umsetzt.

7. Verfahren nach Anspruch 5, **dadurch gekennzeichnet, daß** man 4-Chlorphenylboronsäure mit einer Verbindung der Formel: 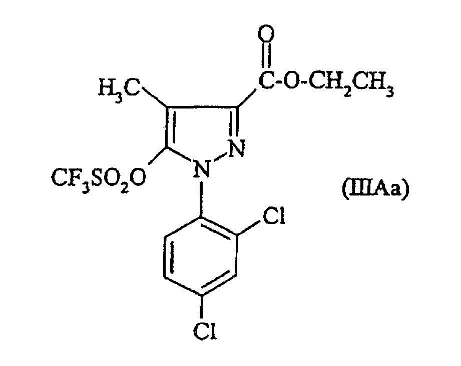 umsetzt.

8. Verfahren nach Anspruch 5, **dadurch gekennzeichnet, daß** man 4-Bromphenylboronsäure mit einer Verbindung der Formel: 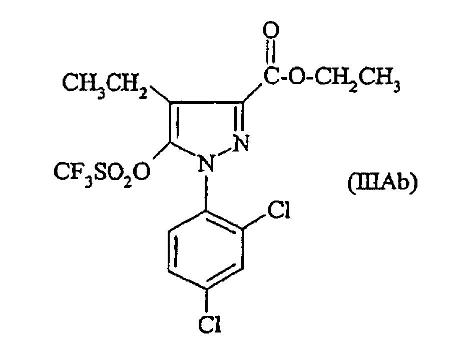 umsetzt.

9. Verfahren nach Anspruch 6, **dadurch gekennzeichnet, daß** man 4-Chlorphenylboronsäure mit einer Verbindung der Formel: 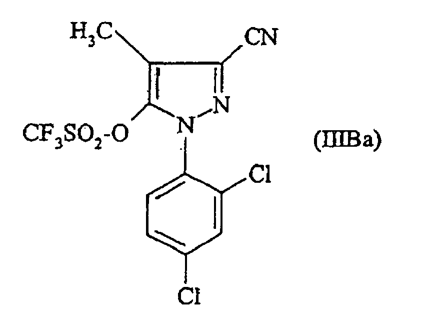 umsetzt.

10. Verfahren nach Anspruch 6, **dadurch gekennzeichnet, daß** man 4-Bromphenylboronsäure mit einer Verbindung der Formel: 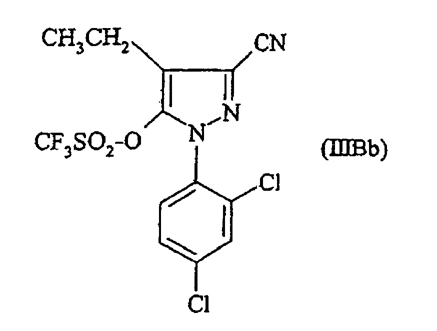 umsetzt.

11. Verbindung der Formel: 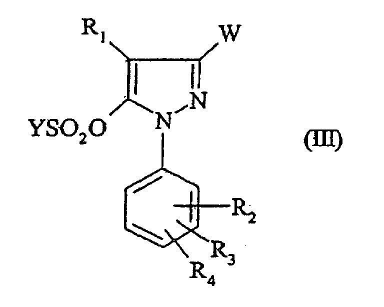 worin:
- W für eine -COOR-Gruppe oder einen -CN-Rest steht;
- Y für eine (C₁-C₄)-Alkylgruppe, eine (C₁-C₄)-Perfluoralkylgruppe oder eine Phenylgruppe, die gegebenenfalls durch eine Methyl-, Chlor- oder Nitrogruppe substituiert ist, steht;
- R₁ für ein Wasserstoff- oder Halogenatom oder eine (C₁-C₄)-Alkylgruppe steht;
- R₂, R₃ und R₄ jeweils unabhängig voneinander für ein Wasserstoff- oder Halogenatom oder eine (C₁-C₄)-Alkyl-, (C₁-C₄)-Alkoxy- oder Trifluormethylgruppe stehen;
- R für eine (C₁-C₄)-Alkyl- oder Benzylgruppe steht.

12. Verbindung nach Anspruch 11 der Formel: 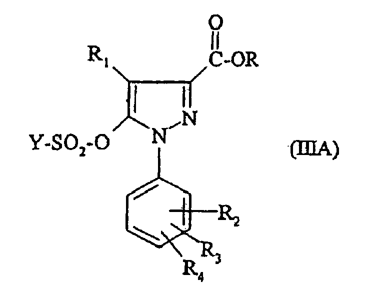 worin:
- Y, R, R₁, R₂, R₃ und R₄ die in Anspruch 11 angegebene Bedeutung besitzen.

13. Verbindung nach Anspruch 11 der Formel: 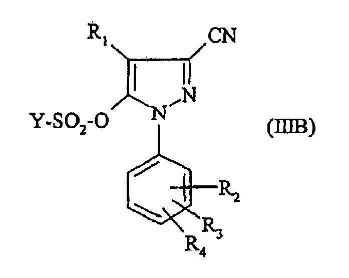 worin:
- Y, R₁, R₂, R₃ und R₄ die in Anspruch 11 angegebene Bedeutung besitzen.

14. Verbindung nach Anspruch 12 der Formel:

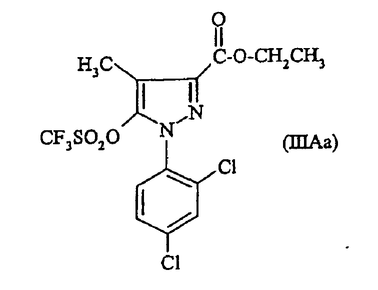

15. Verbindung nach Anspruch 12 der Formel:

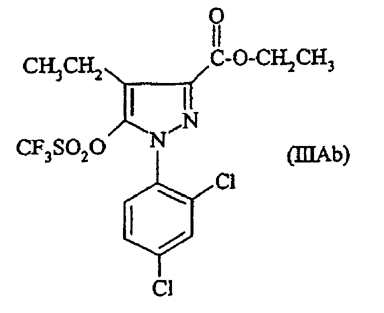

16. Verbindung nach Anspruch 13 der Formel:

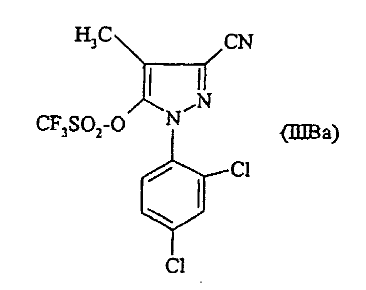

17. Verbindung nach Anspruch 13 der Formel:
